# EUROPEAN PATENT APPLICATION

(11) **EP 4 056 175 A1**
(43) Date of publication of application: **14.09.2022**
(21) Application number: 20885454.7
(22) Date of filing: 06.11.2020
(51) Int. Cl.: A61K 31/045, A61P 25/00, A61P 25/16, A61P 25/28, A61P 27/02, A61P 27/12, A61P 43/00

(54) **METHOD FOR SELECTIVELY KILLING PROTEIN AGGREGATE-CONTAINING CELLS, KIT THEREFOR, THERAPEUTIC DRUG FOR PROTEIN MISFOLDING DISEASES AND DRUG PRODUCT FOR REMOVING PROTEIN AGGREGATES FROM BLOOD PRODUCT**

(30) Priority: 08.11.2019 JP 2019203524; 20.12.2019 JP 2019230543
(71) Applicant: Central Glass Company, Limited, Ube-shi, Yamaguchi 755-0001 (JP)
(72) Inventor: OGAWA Tsuyoshi, Tokyo 101-0054 (JP)
(74) Representative: Manitz Finsterwald Patent- und Rechtsanwaltspartnerschaft mbB
(86) International application number: PCT/JP2020/041522
(87) International publication number: WO 2021/090911

(57) **Abstract**

A method for selectively killing protein aggregate-containing cells, a kit thereof, a therapeutic agent for the protein misfolding disease, and an agent for removing a protein aggregate from a blood derivative are provided. The method for selectively killing cells according to one embodiment acts a fluorine-based alcohol or a compound represented by general formula (1) on cells as a compound for detecting a protein aggregate and selectively kills protein aggregate-containing cells. 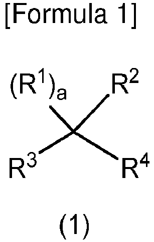 (in general formula (1), a is 0 or 1,
when a is 1, then R¹ is a hydrogen atom and R² is a hydroxyl group, or R¹ is a hydroxyl group and R² is a hydrogen atom, when a is 0, then R² is an oxygen atom forming a double bond together with a carbon atom, R³ is CHₗClₘFₙ, and I is an integer from 0 to 3, m and n are integers from 1 to 3, and I + m + n = 3 is satisfied, R₄ is CHₛClₜFᵤ, and s is an integer from 0 to 3, t and u are integers from 1 to 3, and s + t + u = 3 is satisfied. I + s < 6 is satisfied.)

## Description

### TECHNICAL FIELD

The present invention relates to a method for selectively killing cells containing a protein that can be pathogens of a group of diseases caused by alterations in protein conformation, such as prion disease. Alternatively, the present invention relates to a therapeutic agent for a protein misfolding disease. Alternatively, the present invention relates to an agent for removing protein aggregates from a blood derivative.

### BACKGROUND ART

A protein becomes a functional molecule only after biosynthesis and forming a proper conformation. However, if the proper conformation is not formed, a protein may be fibrotic or aggregated, losing its original function, or gaining new toxicity, blocking various metabolic pathways in the body, and eventually leading to death. These diseases are called protein-misfolding diseases, and the field of neurological diseases includes prion disease, Alzheimer's disease, Parkinson's disease, and Lewy body dementia. The ophthalmologic field includes cataract and age-related macular. Amyloidosis is included in systemic organs.

A prion protein (PrP) is considered a pathogen that causes prion disease, which is a neurodegenerative disease. The prion disease is a zoonotic disease, and sporadic Creutzfeldt-Jakob disease (CJD), inherited Gerstmann-Strasler-Scheinker syndrome (GSS), fatal familial insomnia (FFI), and infectious variant CJD (vCJD) are known in humans. Bovine spongiform encephalopathy (BSE) is known in cattle, scrapie is known in sheep, and deer chronic wasting disease (CWD) is known in deer. The prion disease also develops in cats, minks, and cheetahs, etc. Protein higher-order structural changes from a normal prion protein (PrP^{C}) to an abnormal prion protein (PrP^{Sc}) do not immediately cause prion infection. Although a correlation of these is still controversial, it is clear that PrP^{Sc} is involved in the development of prion disease. However, no fundamental therapy has been found to date. (Non-Patent Literature 1, Non-Patent Literature 2)

In the prion disease, it is often considered that PrP^{Sc} accumulates as protein aggregates inside and outside neurons in the brain, particularly in the central nervous system in the body, and exhibits pathogenicity. PrP^{Sc}has exactly the same amino acid sequence as PrP^{C}, but the conformation of protein is significantly different. PrP^{C} contains a large amount of α-helix structures, while PrP^{Sc}contains a large amount of β-sheet structures. Therefore, PrP^{Sc} becomes a hardly soluble and persistent protein and exhibits resistance to proteolytic enzymes such as protease K. The mechanism of higher-order structural conversion from PrP^{C} to PrP^{Sc} is still unclear. Although it is said that changes in conformation are neurotoxic and destroy nerve cells and the like over time, the details are still unknown. Alzheimer's disease, Parkinson's disease, Lewy body dementia, and the like are also caused by amyloid β protein, tau protein, α-synuclein, and the like as protein aggregates, and are thought to be caused by protein conformation abnormalities (misfolding) as well as prion disease.

Unlike systemic organs, the central nervous system is composed of a group of cells that have completed differentiation, so once a group of proteins undergoing conformational abnormalities accumulates, they are not metabolized and remain in nerve cells.

Therefore, research and development of therapeutic methods, diagnostic methods, and therapeutic methods for the protein misfolding disease are based on a strategy focusing on hardly soluble and persistent protein aggregates that accumulate in a body. Patent Literature 1 discloses a prion disease prevention/therapeutic agent containing a compound composed of an aromatic ring containing a methylene chain or a salt thereof as an active ingredient, and is said to suppress structural conversion of the prion protein. Patent Literature 2 and Patent Literature 3 disclose a compound composed of an aromatic ring as a prion protein structure conversion inhibitor, and it is said that the compound has an action of tightly binding to a normal prion protein and inhibiting structural conversion into an infectious prion protein. These compounds were chemically synthesized by organic chemistry. Patent Literature 4 discloses a naturally-derived abnormal prion protein formation inhibitor that contains high molecular weight polyphenol extracted from a fermented tea as an active ingredient.

In the development strategy of a therapeutic agent, clinical research has been developed in which a drug originally used for the treatment of other diseases is applied to the treatment of the prion disease, and Non-Patent Literature 3 discloses an example using quinacrine, which is an antimalarial drug. Non-Patent Literature 4 discloses an example in which pentosan polysulfate (heparin sulfate), which is a drug for the treatment of interstitial cystitis and arthritis, is used for experimental treatment of prion disease patients. They have not yet been found to have a definite therapeutic effect.

To date, a drug approved for the treatment of protein aggregation diseases includes a therapeutic agent for transthyretin-type amyloidosis (Tafamidis meglumine, manufactured by Pfizer Inc., product name: Vyndaqel). This therapeutic agent has been found to be effective in inhibiting the formation pathway of the protein aggregates and delaying the progression of symptoms.

### CITATION LIST

### PATENT LITERATURE

Patent Literature 1: Japanese Laid Open Patent No. 2018-35099
Patent Literature 2: Japanese Laid Open Patent No. 2009-13126
Patent Literature 3: Japanese Laid Open Patent No. 2010-131717
Patent Literature 4: Japanese Laid Open Patent No. 2009-29752
Patent Literature 5: Japanese Laid Open Patent No. 2009-167128

### NON-PATENT LITERATURE

Non-Patent Literature 1: Hidehiro Mizusawa, Prions and prion diseases, Pharma Medica Vol. 35, No. 2, 67-69 (2017)
Non-Patent Literature 2: Kentaro Sahashi, Targeted therapeutic approaches for aggregation-prone proteins, SEITAI NO KAGAKU, Vol. 67, No. 4, 296-302 (2016)
Non-Patent Literature 3: Tatsuo Yamada, Katsumi Doh-ura, Qinacrine treatment for Creutzfeldt-Jakob disease, H15 Research report of the research on prion disease and slow virus infection in the specific disease countermeasure research project of the Ministry of Health, Labor and Welfare Scientific Research Grant, (2004) p. 113-124
Non-Patent Literature 4: Tsuboi Y, Doh-Ura K, Yamada T. et al. Continuous intraventricular infusion of pentsan polysulfate: clinical trial against prion diseases. Neuropathology 2009; 29: 362-636

### SUMMARY OF INVENTION

### TECHNICAL PROBLEM

One embodiment of the present invention has been made in view of the above, and it is an object of the present invention to provide a method that specifically acts on cells containing protein aggregates and selectively kills protein aggregate-containing cells, and a kit thereof. Alternatively, one embodiment of the present invention is directed to providing a therapeutic agent for a protein misfolding disease. Alternatively, one embodiment of the present invention is directed to providing an agent for removing protein aggregates from a blood derivative.

### SOLUTION TO PROBLEM

As a result of extensive studies by the present inventors, it has been found that when 1,1,1,3,3,3-hexafluoro-2-propanol (HFIP), which is a fluorine-based alcohol, is contacted with protein aggregates, the HFIP acts specifically on the protein aggregates to act as a molecular structure-modifying agent, thereby changing the conformation of the protein aggregates. Then, it has been newly found that the HFIP selectively kills protein aggregate-containing cells.

The present invention includes the following aspects.
[1] A method for selectively killing protein aggregate-containing cells by acting a fluorine-based alcohol or a molecular structure-modifying agent containing a compound represented by the following general formula (1) on cells. 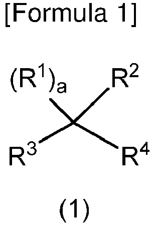 (In general formula (1), a is 0 or 1,
   when a = 1, then R¹ is a hydrogen atom and R² is a hydroxyl group, or R¹ is a hydroxyl group and R² is a hydrogen atom,
   when a = 0, then R² is an oxygen atom forming a double bond together with a carbon atom,
   R³ is CHₗClₘFₙ, and I is an integer from 0 to 3, m and n are integers from 1 to 3, and I + m + n = 3 is satisfied,
   R⁴ is CHₛClₜFᵤ, and s is an integer from 0 to 3, t and u are integers from 1 to 3, and s + t + u = 3 is satisfied,
   I + s < 6 is satisfied.)
[2] A method for selectively killing cells in which the fluorine-based alcohol is represented by a general formula RfCH₂OH, or RfRf'CHOH, Rf and Rf' present a perfluoroalkyl group having 1 to 10 carbon atoms, and Rf and Rf' are different from each other or the same.
[3] A method for selectively killing cells in which the fluorine-based alcohol is 1,1,1,3,3,3-hexafluoro-2-propanol.
[4] A method for selectively killing cells in which a concentration of the fluorine-based alcohol or a compound represented by general formula (1) when treating the cells is in a range from 10 Pm to 100 Mm.
[5] A kit for selectively killing cells containing a fluorine-based alcohol or a compound represented by the following general formula (1) as a compound for detecting a protein aggregate. 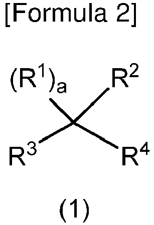 (In general formula (1), a is 0 or 1,
   when a = 1, then R¹ is a hydrogen atom and R² is a hydroxyl group, or R¹ is a hydroxyl group and R² is a hydrogen atom,
   when a = 0, then R² is an oxygen atom forming a double bond together with a carbon atom,
   R³ is CHₗClₘFₙ, and I is an integer from 0 to 3, m and n are integers from 1 to 3, and I + m + n = 3 is satisfied,
   R⁴ is CHₛClₜFᵤ, and s is an integer from 0 to 3, t and u are integers from 1 to 3, and s + t + u = 3 is satisfied,
   I + s < 6 is satisfied.)
[6] The fluorine-based alcohol of the kit for selectively killing cells is presented by a general formula RfCH₂OH or RfRf'CHOH,
   Rf and Rf' present a perfluoroalkyl group having 1 to 10 carbon atoms, and
   Rf and Rf' are different from each other or the same.
[7] The fluorine-based alcohol of the kit for selectively killing cells is 1,1,1,3,3,3-hexafluoro-2-propanol.
[8] The kit for selectively killing protein aggregate-containing cells, wherein a concentration of the fluorine-based alcohol or a compound represented by general formula (1) when treating the cells is in a range from 10 Pm to 100 Mm.
[9] A therapeutic agent for a protein misfolding disease including a fluorine-based alcohol or a compound represented by the following general formula (1). 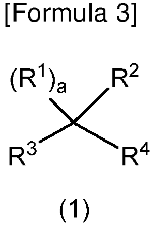 (In general formula (1), a is 0 or 1,
   when a = 1, then R¹ is a hydrogen atom and R² is a hydroxyl group, or R¹ is a hydroxyl group and R² is a hydrogen atom,
   when a = 0, then R² is an oxygen atom forming a double bond together with a carbon atom,
   R³ is CHₗClₘFₙ, and I is an integer from 0 to 3, m and n are integers from 1 to 3, and I + m + n = 3 is satisfied,
   R⁴ is CHₛClₜFᵤ, and s is an integer from 0 to 3, t and u are integers from 1 to 3, and s + t + u = 3 is satisfied,
   I + s < 6 is satisfied.)
[10] The fluorine-based alcohol of the therapeutic agent for the protein misfolding disease is represented by a general formula RfCH₂OH, or RfRf'CHOH, Rf and Rf' present a perfluoroalkyl group having 1 to 10 carbon atoms, and Rf and Rf' are different from each other or the same.
[11] The fluorine-based alcohol of the therapeutic agent for the protein misfolding disease is 1,1,1,3,3,3-hexafluoro-2-propanol.
[12] An agent for removing a protein aggregate from a blood derivative containing a fluorine-based alcohol or a compound represented by the following general formula (1): 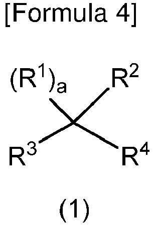 (In general formula (1), a is 0 or 1,
   when a = 1, then R¹ is a hydrogen atom and R² is a hydroxyl group, or R¹ is a hydroxyl group and R² is a hydrogen atom,
   when a = 0, then R² is an oxygen atom forming a double bond together with a carbon atom,
   R³ is CHₗClₘFₙ, and I is an integer from 0 to 3, m and n are integers from 1 to 3, and I + m + n = 3 is satisfied,
   R⁴ is CHₛClₜFᵤ, and s is an integer from 0 to 3, t and u are integers from 1 to 3, and s + t + u = 3 is satisfied,
   I + s < 6 is satisfied.)
[13] In the agent for removing the protein aggregate from the blood derivative, the fluorine-based alcohol is presented by a general formula RfCH₂OH, or RfRf'CHOH, Rf and Rf' present a perfluoroalkyl group having 1 to 10 carbon atoms, and Rf and Rf' are different from each other or the same.
[14] In the agent for removing the protein aggregate from the blood derivative, the fluorine-based alcohol is 1,1,1,3,3,3-hexafluoro-2-propanol.

### ADVANTAGEOUS EFFECTS OF INVENTION

According to one embodiment of the present invention, by using a molecular structure-modifying agent that alters a protein conformation with respect to protein aggregates that accumulated in humans and animals, it is possible to specifically act on protein aggregate-containing cells and to selectively kill the protein aggregate-containing cells. Alternatively, according to one embodiment of the present invention, the molecular structure-modifying agent can be used as a therapeutic agent for the protein misfolding disease by altering the protein conformation for the protein aggregates accumulated in humans and animals. Alternatively, according to one embodiment of the present invention, even when protein aggregates are contained in a blood derivative, protein aggregates can be removed to provide a safe blood derivative.

### BRIEF DESCRIPTION OF DRAWINGS

FIG. 1 is a photograph of cell culture plates tested for sensitivity at various concentrations of HFIP to Neuro 2a (N2a) cells producing PrP^{C} and ScNeuro 2a (ScN2a) cells producing PrP^{Sc} in cultured cells derived from mouse neuroblastoma.
FIG. 2 is a result of an evaluation of the resistance of N2a cells and ScN2a cells after cell culture to protease K by Western blotting under the condition of the presence or absence of HFIP addition.
FIG. 3 is a schematic diagram showing an inspection apparatus 100 according to one embodiment of the present invention.

### DESCRIPTION OF EMBODIMENTS

A compound that alters the molecular structure of protein aggregates of humans and animals of the present invention is a fluorine-based alcohol or a compound represented by the following general formula (1).

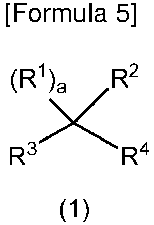

In the above general formula (1), a is 0 or 1, and when a = 1, R¹ is a hydrogen atom and R² is a hydroxyl group. Alternatively, R¹ is a hydride group and R² is a hydride atom. When a = 0, then R² is an oxygen atom forming a double bond together with a carbon atom. R³ is CHₗClₘFₙ, I is an integer from 0 to 3, m and n are integers from 1 to 3, and l + m + n = 3 is satisfied. R⁴ is CHₛClₜFᵤ, and s is an integer from 0 to 3, t and u are integers from 1 to 3, and s + t + u = 3 is satisfied. I + s < 6 is satisfied.

In one embodiment, the molecular structure-modifying agent contains a fluorine-based alcohol or a compound represented by the above general formula as an active ingredient. In this specification, a "molecular structure-modifying agent" includes a compound that specifically acts on a protein containing a large amount of β-sheet structures to unfold its molecular structure from the β-sheet structure to induce the formation of an α-helix structure, and is suitably available for detection of protein aggregates and simple screening and diagnosis of protein misfolding diseases including a neurodegenerative disease. In one embodiment, the molecular structure-modifying agent contains two or more kinds selected from the fluorine-based alcohol and the compound represented by the above general formula.

In one embodiment, the compound represented by the above general formula (1) may be selected from the compound group represented by the following compounds 1 to 3.

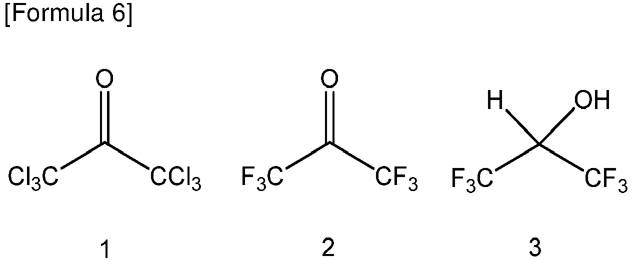

The fluorine-based alcohol of the present invention is represented by the following general formula (2) or (3).

RfCH₂OH ... (2)

RfRf'CHOH ... (3)

In the formulae, Rf, Rf' present a perfluoroalkyl group having 1 to 10 carbon atoms.

Among the compounds represented by the general formula (2) or (3), particularly, 1,1,1,3,3,3-hexafluoro-2-propanol (hereinafter, sometimes referred to as HFIP), 2,2,2-trifluoroethanol, and 2,2,3,3,3-pentafluoro-1-propanol can be exemplified. In particular, HFIP is suitable as a compound that alters the molecular structure of protein aggregates. In one embodiment, the molecular structure-modifying agent may contain at least one of the compounds represented by the general formulas (1), (2) or (3) and a solvent.

Examples of the solvent capable of diluting the compound represented by the general formulas (1), (2), or (3) include, but are not limited to, water, saline, Ringer's solution, phosphate buffered saline (abbreviation PBS), methanol, ethanol, isopropanol, acetone, toluene, dimethyl sulfoxide, and the like. In one embodiment, the molecular structure-modifying agent includes a compound represented by the general formula (1), (2), or (3) and a solvent described above. For example, since HFIP is a colorless transparent liquid having a melting point of -3.3°C and a boiling point of 58.6°C and is soluble in most solvents, HFIP can be adjusted to an arbitrary concentration by diluting with the solvent.

The concentration of the fluorine-based alcohol or the compound represented by the general formula (1) when acting on the protein aggregate is preferably 10 pM or more and 100 mM or less. The time for causing the fluorine-based alcohol or the compound represented by the general formula (1) to act on the protein aggregates is not particularly limited.

In one embodiment, a compound in which a hydroxy group in a fluorine-based alcohol or a compound represented by the general formula (1) is protected with a protecting group may be used together with the fluorine-based alcohol or the compound represented by the general formula (1), or instead of the fluorine-based alcohol or the compound represented by the general formula (1). By this protective group, decomposition of a fluorine-based alcohol or a compound represented by the general formula (1) is suppressed in a process until it acts with protein aggregates, and this compound may be converted into a fluorine-based alcohol or a compound represented by the general formula (1) when it acts with protein aggregates. Examples of such a protecting group include a functional group commonly used as a protecting group for a hydroxy group (see, for example, "Protecting Group Chemistry" Jeremy Robertson, published by Oxford University Press), a sugar chain, a peptide, and the like. Examples of the functional group used as the protecting group include, but are not limited to, an alkoxyalkyl group (for example, a methoxymethyl group, an ethoxyethyl group), an acetal-based functional group such as a 2-tetrahydropyranyl group, an acyl-based functional group such as an alkanoyl group (e.g., an acetyl group) or an aroyl group (e.g., an arylmethyl group such as a benzyl group or a benzoyl group), or a silyl ether-based functional group such as an alkylsilyl group (e.g., a trimethylsilyl group, a triethylsilyl group or a tert-butyldimethylsilyl group).

### [Method for selectively killing protein aggregate-containing cells]

The fluorine-based alcohol or the molecular structure-modifying agent containing a compound represented by the general formula (1) of the present invention can selectively kill protein aggregate-containing cells in a patient such as a patient with a protein misfolding disease, particularly a neurodegenerative disease, and can further make a kit for selectively killing protein aggregate-containing cells by being contained as an active ingredient at a pharmaceutically acceptable concentration. The pharmaceutically acceptable concentration here is the same as the concentration at which the molecular structure of a protein aggregate is altered, and specifically, 10 pM or more and 100 mM is preferred. In one embodiment, a kit for selectively killing protein aggregate-containing cells includes HFIP and the solvent described above.

In this specification, a protein misfolding disease in the nervous system can be exemplified by prion disease such as sporadic Creutzfeldt-Jakob disease (CJD), inherited Gerstmann-Strasler-Scheinker syndrome (GSS), fatal familial insomnia (FFI), iatrogenic or dietary variant CJD (vCJD), bovine spongiform encephalopathy (BSE), sheep scrapie, deer chronic wasting disease (CWD), and prion diseases in animals such as cats, minks, cheetahs that may develop prion disease. Other examples include neurodegenerative diseases included in the spectrum of Alzheimer's disease, Parkinson's disease, Lewy body dementia, frontotemporal lobe degeneration (FTLD), diseases such as amyotrophic lateral sclerosis, Huntington's chorea, and polyglutamine disease. In the ophthalmologic field, cataract, age-related macular, and the like can be exemplified. Amyloidosis is included in systemic organs.

In neurodegenerative diseases, it is considered that proteins showing a normal conformation undergo higher-order structural changes to proteins with abnormal conformation, particularly in the central nervous system in the body, and this accumulates as protein aggregates inside and outside nerve cells in the brain and exhibits pathogenicity. In sporadic and infectious prion diseases, the amino acid sequence of the protein does not change in this process, only its conformation. Normal proteins contain a large amount of α-helix structures as secondary structures, whereas proteins with abnormal conformation contain a large amount of β-sheet structures, resulting in hardly soluble and persistent proteins and exhibiting resistance to proteolytic enzymes such as protease K.

The fluorine-based alcohol or the compound represented by the general formula (1) contained in the molecular structure-modifying agent of the present invention, especially HFIP, acts specifically on a β-sheet structure of a protein to unfold its molecular structure from the β-sheet structure to induce the formation of an α-helix structure, and can be an effective molecular structure-modifying agent against protein aggregates accumulated in a body, particularly in central nervous tissue, in neurodegenerative diseases. Since HFIP is a low molecular compound with a molecular weight of 168 and the molecular size allows it to pass through the blood-brain barrier which controls the material exchanges between blood and brain tissue fluids (generally, molecules with a molecular weight greater than 500 cannot pass through), it is thought to migrate into the brain and function as a molecular structure-modifying agent.

In one embodiment, protein aggregate-containing cells of the present invention are cells derived from organs or living tissues suspected of having been infected with a protein misfolding disease, such as, but not limited to, brain, medulla oblongata, amygdala, nerve connection, spleen, heart, liver, lung, eye, placenta, testis, lymphoid tissue, muscle tissue, etc., or nerve cells, blood cells, muscle cells, and cells derived from body fluid such as blood (including fluid components of plasma, serum), cerebrospinal fluid (spinal fluid), and urine. For detecting the presence or absence of the protein aggregate-containing cells, blood, cerebrospinal fluid, and the like are preferable because they are specimens obtained by relatively low invasion. Amygdala, nerve connection, etc. are tissues that are easier to collect when compared with the brain, making them suitable for the diagnosis of industrial animals.

Biological samples infected with the protein misfolding disease include protein aggregate-containing cells, which can be used for simple screening or diagnosis of the protein misfolding disease by utilizing the difference in sensitivity between these cells to fluorine-based alcohol or compounds of general formula (1). In one embodiment, for example, cells suffering from the protein misfolding disease lead to cell death depending on the concentration of the fluorine-based alcohol or the compound represented by general formula (1) as compared to normal cells. To increase the detection sensitivity of the protein aggregates, biological samples taken from biological tissues may be cultured outside the body and then used. Since a suitable biological sample is different for proteins to be detected, it is not particularly limited. For example, it is said that PrP^{Sc} is present in spinal fluid, and amyloid beta of Alzheimer's disease and the like is also present in blood.

The detection of protein aggregates of the present invention can be used not only for simple screening and diagnosis of the protein misfolding disease but also for tests for preventing infection by blood for transfusion, blood derivatives, spinal fluid, organs for transplantation, and the like. Currently, the blood donors have restrictions, such as the history of staying abroad and disease history of the blood donors, on the testing of prion infections in blood for transfusions.

The test for altering the molecular structure of the protein aggregates using the fluorine-based alcohol or the compound represented by the general formula (1) of the present invention is not particularly limited as long as it is a method capable of evaluating proteins with a normal molecular structure and proteins with an abnormal molecular structure, respectively.

In one embodiment, when the above cells are used as biological samples, it is possible to detect the presence or absence of a cell in which the protein aggregates have accumulated due to the difference in sensitivity to the molecular structure-modifying agent of the present invention between the normal cell and cells in which protein aggregates have accumulated. For example, by acting the molecular structure-modifying agent of the present invention on cells for a predetermined time, and then evaluating living or dead cells by cell staining, it is possible to detect the presence or absence of cells in which protein aggregates have accumulated.

In one embodiment, when the above tissues or cells are used as biological samples, the presence or absence of the tissues or cells in which the protein aggregates have accumulated can be detected by the difference in sensitivity to proteolytic enzymes between the protein with the normal structure and the protein aggregate with respect to the molecular structure-modifying agent of the present invention. For example, when cytolytic solution is treated with proteolytic enzymes, the protein aggregates do not undergo degradation by proteolytic enzymes, and a band of the protein aggregates is detected by electrophoresis. On the other hand, when cytolytic solution is treated with the molecular structure-modifying agent of the present invention and then treated with proteolytic enzymes, the protein aggregates altered by the molecular structure-modifying agent are decomposed by proteolytic enzymes, and the band of the protein aggregates is diluted or disappeared by electrophoresis. Therefore, it is possible to detect the presence or absence of tissues or cells in which the protein aggregates have accumulated by the difference in sensitivity to proteolytic enzymes before and after the treatment of the molecular structure-modifying agent of the present invention. Even if the protein from which the protein aggregates are formed is unknown, the protein can be identified, for example, by mass spectrometry using a shotgun method or by a combination of two-dimensional electrophoresis and mass spectrometry.

### [Inspection apparatus]

In one embodiment, it is possible to provide an inspection apparatus including the fluorine-based alcohol or the compound represented by the general formula (1) according to the present invention described above in a detection unit.

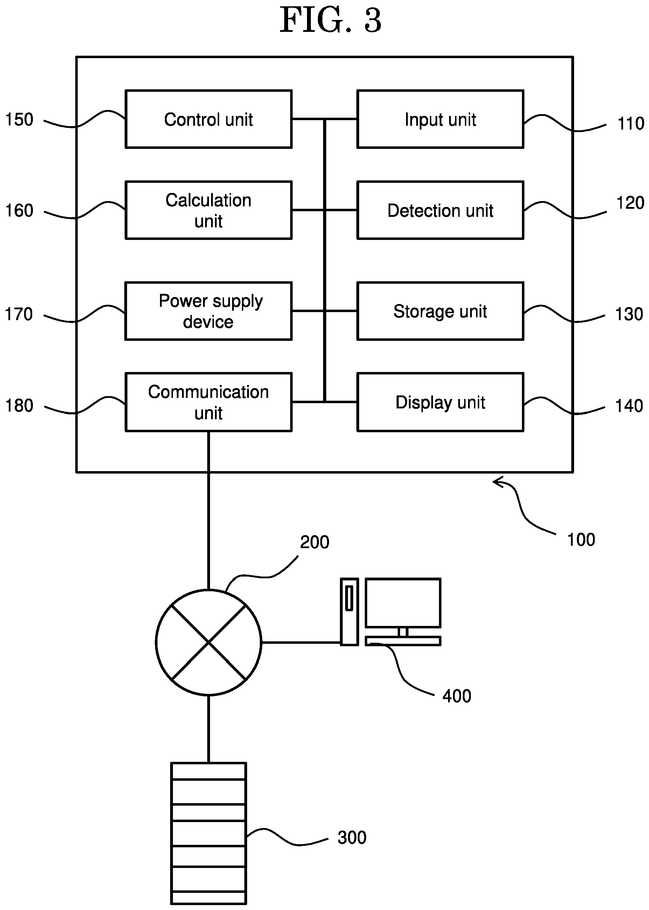

FIG. 3 is a schematic diagram showing an inspection apparatus 100 according to one embodiment of the present invention. The inspection apparatus 100 includes, but is not limited to, an input unit 110, a detection unit 120, a storage unit 130, a display unit 140, a control unit 150, a calculation unit 160, a power supply device 170, and a communication unit 180, for example. The inspection apparatus 100 may be connected to a server 300 via a network 200.

The input unit 110 is, for example, a device for inputting information such as an inspection target, an inspection condition, and the like to the inspection apparatus 100, and may be configured by an input device such as a well-known keyboard, mouse, or touch panel. The information to be examined may be, for example, names of the above cells or tissues, names of patients, IDs, or the like. The inspection condition may be a condition related to an examination such as names and concentrations of the fluorine-based alcohol or the compound represented by the general formula (1) according to the present invention, inspection dates and time, cell numbers, and the like.

The detection unit 120 is, for example, a device that detects a state of cells or protein aggregates by adding the fluorine-based alcohol or the compound represented by general formula (1) according to the present invention to cells or the like to be examined. In one embodiment, the detection unit 120 may contain the fluorine-based alcohol or the compound represented by general formula (1) according to the present invention. For example, the detection unit 120 may supply the fluorine-based alcohol or the compound represented by the general formula (1) according to the present invention to cells or the like placed in a detection container to detect a state of the cells or protein aggregates. In this specification, the detection unit 120 shows a means for performing a series of processes for detecting the state of the cells or protein aggregates by supplying the fluorine-based alcohol or the compound represented by the general formula (1) according to the present invention, and it may include performing a supply of the fluorine-based alcohol or the compound represented by the general formula (1) according to the present invention by a person and detecting the state of the cells or protein aggregates by the detection unit 120. The detection unit 120 may include an electrophoresis device for detecting the state of the protein agglomerates. In order to detect the state of the cells or protein aggregates, the detection unit 120 may include an imaging device. For example, a known CCD image sensor or a CMOS image sensor can be used as the imaging device, and a detailed description thereof will be omitted.

The storage unit 130 includes a main storage device and an auxiliary storage device. Since a known memory can be used for the main memory device, a detailed description will be omitted. A known hard disk or a solid-state drive (SSD) can be used as the auxiliary memory device, and a detailed description will be omitted. The storage unit 130 can store operating systems and application software for the inspection apparatus 100, and data such as detection results. The auxiliary memory device is not essential, and for example, in the case where the inspection apparatus 100 is connected to the server 300 via the network 200, the application software and the data of detection results and the like may be stored in the server 300.

The display unit 140 is a device for displaying information for operating the inspection apparatus 100, detection results, and the like, and since a known display can be used, detailed descriptions thereof are omitted.

The control unit 150 is comprised of a central processing unit and a program for controlling the inspection apparatus 100. The control unit 150 includes, for example, an operating system, application software, or a module.

The calculation unit 160 performs an operation on the basis of the information to be inspected and the inspection condition input from the input unit 110, and the state of the cells and the protein aggregates obtained by the detection unit 120, and provides an inspection result. The calculation unit 160 includes an application software or module used for calculation.

Since the power supply device 170 is a device for supplying a power source from the outside to the inspection apparatus 100 and it is possible to use a known power supply device or a battery, a detailed description thereof will be omitted.

The communication unit 180 is a device for the inspection apparatus 100 to communicate with an external apparatus by cables or wirelessly. The communication unit 180 can be connected to the server 300 or other devices (not shown) via the network 200 such as a well-known local area network (LAN) or the internet.

In one embodiment, the inspection apparatus 100 may detect the state of the cells to be examined or the state of the protein aggregates after the fluorine-based alcohol or the compound represented by general formula (1) according to the present invention is acted on by the detection unit 120, and compare the result with the control data or the standard value stored in the storage unit 130 or the server 300, thereby evaluating the risk of developing the protein misfolding disease and the degree of progress of the disease.

In one embodiment, the inspection apparatus 100 may compare the inspection result at the time of inspection (first time point) and the previous (second time point) inspection result stored in the storage unit 130 or the server 300 to evaluate the risk of developing the protein misfolding disease and the degree of progression of the disease.

In one embodiment, the inspection apparatus 100 may cause a neural network to perform a machine-learning process using teacher data stored in the storage unit 130 or the server 300, and may use the learned neural network to evaluate the risk of developing the protein misfolding disease and the degree of progression of the disease based on the data to be inspected detected by the detection unit 120. In this case, the teacher data includes a predetermined input data (such as a detection result) related to each sample collected from a plurality of samples and output data indicating the possibility in which the organism from which the sample has been collected may be a protein misfolding disease.

In one embodiment, the inspection apparatus 100 may output the evaluation results by a printer via the communication unit 180, and may transmit the evaluation results from the communication unit 180 to a terminal 400 for electronic medical records or medical professionals via the network 200.

### [Therapeutic agent for protein misfolding disease]

In one embodiment, the compound that alters the molecular structure of the protein aggregates of humans and animals of the present invention described above can be used as a therapeutic agent for the protein misfolding disease. The therapeutic agent for the protein misfolding disease contains the fluorine-based alcohol or the compound represented by the above general formula (1).

In one embodiment, the therapeutic agent for the protein misfolding disease may be a form which controls pharmacokinetics in a body by using a form in which the fluorine-based alcohol or the compound represented by the above general formula (1) is bound to lipids such as cholesterol, or a preparation in which the fluorine-based alcohol or the compound represented by the above general formula (1) is supported on a carrier or support such as a liposome or a drug sustained-release material. In one embodiment, these therapeutic agents for the protein misfolding disease may be labeled with a label such as fluorescent substances, isotopes, enzymes, and the like to evaluate their binding activity. For detection of the labeled material, known equipment and means such as radiation (including PET, positron emission tomography), fluorescence, light emission, and the like can be used.

In one embodiment, the therapeutic agent for the protein misfolding disease can be administered to humans, cows, sheep, deer, etc., which are mammals suffering from zoonosis. The form of administration may be either oral or parenteral, and systemic or local administration via transdermal, intradermal, subcutaneous, muscle, abdominal cavity, arterial, venous, rectal, spinal cord, medullary cavity, nasal cavity, eye, sublingual, brain, and the like may be selected.

For example, an injection agent or infusion solution in which a therapeutic agent for the protein misfolding disease is diluted and dissolved in water (water for injection), physiological saline solution, glucose solution, buffer solution, or the like can be performed by an administration method such as an injection or infusion into transdermal, intradermal, subcutaneous, muscle, abdominal cavity, artery, vein, rectum, eye, spinal cord, medulla oblongata, spinal cavity, dura matter, brain, or the like. Suitable dispersing and suspending agents for the injection agent and infusion may be used.

In one embodiment, for example, a cream, an ointment, an aerosol, a gel, an eye-drop, or the like containing the therapeutic agent for the protein misfolding disease, or a solid such as a tablet, a capsule, a granule, a tablet, a suppository, a patch and a cataplasm, or a form in which the therapeutic agent for the protein misfolding disease is carried on a carrier may be used. It is also possible to select a form that a catheter, a programmed small pump, an osmotic pump, or the like is placed in the brain tissue of a mammal or a disease site in the body, and the therapeutic agent for the protein misfolding disease is locally administered.

In one embodiment, the therapeutic agent for the protein misfolding disease can be provided as an injection agent.

The therapeutic agent for the protein misfolding disease preferably includes a compound in which the fluorine-based alcohol is represented by the general formula RfCH₂OH, or RfRf'CHOH, Rf and Rf' present a perfluoroalkyl group having 1 to 10 carbon atoms, and Rf and Rf' are different each other or the same. In one embodiment, the therapeutic agent for the protein misfolding disease is preferably HFIP.

In one embodiment, the injection agent of the therapeutic agent for the protein misfolding disease can set a dosage per one time in a range which exhibits a therapeutic effect and is pharmaceutically acceptable, and for example, 1 µg to 100 mg can be contained in 1 vial. The injection agent may be provided in a vial containing only the fluorine-based alcohol or the compound represented by the above general formula (1) or may be provided as an injection agent diluted with a pharmaceutically acceptable solvent. Alternatively, it may be provided in a vial containing the fluorine-based alcohol or the compound represented by the above general formula (1) and a pharmaceutically acceptable additive agent, and may be provided as an injection agent in which the fluorine-based alcohol or the compound represented by the above general formula (1) and the pharmaceutically acceptable additive agent are dissolved in the solvent.

For example, a known solvent such as water (water for injection), physiological saline, Ringer's solution, glucose injection solution, or a medically acceptable organic solvent can be used as the pharmaceutically acceptable solvent. The pharmaceutically acceptable additive agent may include, for example, stabilizers, excipients, lubricants, dissolution aids, emulsifiers, pH-adjusting agents, painless agents, antiseptic agent, antioxidants, preserving agent, colorants, various salt compounds, and the like. In one embodiment, the injection agent may include an adjuvant.

A method for producing the therapeutic agent for the protein misfolding disease is not particularly limited, and can be produced by a known method for producing an injection agent.

For example, a method of administration is such that the fluorine-based alcohol or the compound represented by the above general formula (1) is injected intravenously in a state in which it is dissolved in the pharmaceutically acceptable solvent. In one embodiment, the therapeutic agent for the protein misfolding disease may be administered to humans, cows, sheep, deer, and the like at a medically acceptable concentration. Although these cannot be generally determined because they differ depending on the mammal, age, body weight, time of symptom, and the like of the subject to be administered, for example, the concentration of the therapeutic agent for the protein misfolding disease may be adjusted so that the dosage per one time is several µg to several hundred mg per kg of body weight, for example, 1 µg to 100 mg, and it may be administered several times per day to several times per week while observing the clinical course.

The fluorine-based alcohol or the compound represented by the above general formula (1), particularly HFIP, is a low molecular compound with a molecular weight of 168, and when administered by intravenous injection, it is migrated into the brain because it has a molecular size that allows it to pass through the blood-brain barrier which controls the material exchanges between blood and brain tissue fluids (generally, molecules with a molecular weight greater than 500 cannot pass through). It is considered that the fluorine-based alcohol or the compound represented by the above general formula (1) specifically acts on the β-sheet structure of the protein aggregates accumulated in the central nervous system, unfolds its molecular structure from the β-sheet structure to induce it into the α-helix structure, and exhibits a therapeutic effect on the protein misfolding disease.

The therapeutic agent for the protein misfolding disease can be expected to have a therapeutic effect on diseases such as sporadic Creutzfeldt-Jakob disease (CJD), inherited Gerstmann-Strasler-Scheinker syndrome (GSS), fatal familial insomnia (FFI), iatrogenic or dietary variant CJD (vCJD), bovine spongiform encephalopathy (BSE), sheep scrapie, deer chronic wasting disease (CWD), prion diseases in animals such as cats, minks, cheetahs that may develop prion disease, neurodegenerative diseases included in the spectrum of Alzheimer's disease, Parkinson's disease, Lewy body type dementia, frontotemporal lobe degeneration (FTLD), diseases such as amyotrophic lateral sclerosis, Huntington's chorea, and polyglutamine disease, and diseases in the ophthalmologic field such as cataracts, age-related macular, and systemic organ diseases such as amyloidosis.

The therapeutic agent for the protein misfolding disease of one embodiment may be administered to a mammal suspected of having the disease to suppress the onset of symptoms or may be used as a preventive measure against infection of the disease.

The therapeutic agent for the protein misfolding disease of one embodiment may also be used in combination with other drugs. The target disease is not particularly limited as long as it is a drug used in the nervous system and systemic protein misfolding diseases. For example, quinacrine, pentosan polysulfate, or the like may be used in combination in the case of prion disease of the nervous system. These may be administered in one species or in a combination of two or more species.

### [Agent for removing protein aggregate from blood derivative]

In one embodiment, the compound that alters the molecular structure of the protein aggregate of a human and an animal of the present invention described above can be used to remove the protein aggregate contained in a blood derivative (including the case where the compound is used prophylactically in view of the possibility that the protein aggregate is contained in the blood derivative). As a conventional method of removing PrP^{Sc}, which is a protein aggregate, a method of removal by adsorbing PrP^{Sc} using a filter or carrier has been proposed. For example, Patent Literature 5 discloses a filter with a heteromorphic cross-sectional fiber nonwoven fabric and a carrier coated with a polymer composed of three units composed of a unit derived from a hydrophobic polymerizable monomer of 20 mol% or more and 40 mol% or less, a unit derived from a polymerizable monomer containing 5 mol% or more and 13 mol% or less of a basic nitrogen-containing portion, and as residue of these, a unit derived from a polymerizable monomer containing a protonic neutral hydrophilic portion. However, a method for inactivating the protein aggregate contained in the blood derivative has not been reported.

In one embodiment, the agent for removing the protein aggregate contains the fluorine-based alcohol or the compound represented by the above general formula (1). For example, the protein aggregate can be inactivated by adding HFIP in the blood derivative at a concentration range of 0.1 ppm to 5000 ppm. Therefore, even if the protein aggregate is mixed in the blood derivative, the protein aggregate removing preparation according to the present embodiment can inactivate the protein aggregate and provide a safe blood derivative without risk of infection.

In one embodiment, a method of inactivating the protein aggregate can be provided by using a structure including the fluorine-based alcohol or the compound represented by the above general formula (1) and a support medium that holds the fluorine-based alcohol or the compound represented by the above general formula (1). For example, a structure obtained by supporting or impregnating the fluorine-based alcohol or the compound represented by the above general formula (1) on a support medium such as a filter or a carrier is contacted with the blood derivative to inactivate the protein aggregate in the blood derivative. The filter and carrier used for the support medium may be employed from materials known in the field, and examples thereof include natural polymers such as cellulose, synthetic polymers such as polyolefins, polyesters, polyether sulfones, and Teflon (registered trademark), and inorganic materials such as silica, alumina, and zeolite. The shape of the structure is not particularly limited and may be, for example, a shape such as a membrane (film), a mesh, a nonwoven fabric, a hollow fiber, a sponge, a powder, or fine particles.

### [Examples]

Hereinafter, the present invention will be described in more detail by examples, and the present invention is not limited to the following examples as long as it does not exceed the gist thereof.

<Molecular structure-modifying agent>

HFIP (Central Glass Co., Ltd., purity 99% or more) was used as a compound that alters the molecular structure of the protein aggregate.

### <Mouse neuroblastoma-derived cells>

Two cell lines (ATCC) of a Neuro 2a (N2a) cell producing a normal prion protein (PrP^{C}) and ScNeuro 2a (ScN2a) cell continuously producing abnormal prion protein (PrP^{Sc}) were prepared in cultured cells derived from mouse neuroblastoma. The two cell lines were cultured in Eagle's minimal essential medium (E-MEM) (FUJIFILM Wako Pure Chemical Corporation) containing 10% FBS (fetal bovine serum) and 100 Units/ml Penicillin, 100 µg/ml Streptomycin under humidified, 37°C, 5% CO₂ conditions. A cell culture dish (AGC TECHNO GLASS CO., LTD.) and a cell culture microplate (6 wells or 24 wells, AGC TECHNO GLASS CO., LTD.) were used as cell culture vessels.

A 24-well plate for cell culture was seeded with 1 × 10⁵ of cells per well, and N2a cells and ScN2a cells were incubated in E-MEM at 37°C under 5% CO₂ for 2 days, respectively.

### [Example 1]

In the assessment of sensitivity to HFIP described above, various mediums with a final concentration of HFIP in the medium of 0 mM, 10 mM, 20 mM, 25 mM, 30 mM, and 40 mM were used. "M" presents the molar concentration (mol/L). These were added to ScN2a cells and cell cultures were performed for 24 hours.

### [Comparative Example 1]

The procedure was carried out in the same manner as in Example 1, except that the cells used were changed to N2a cells.

### <Assessment of sensitivity to HFIP>

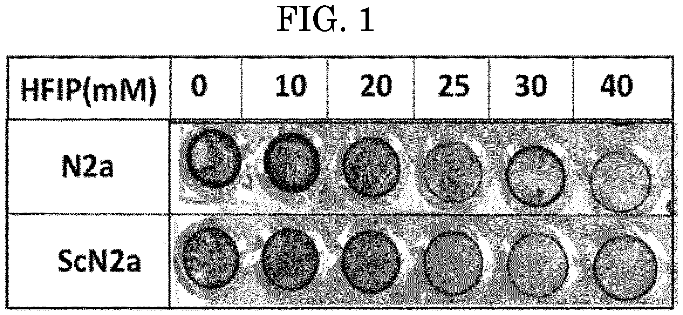

Assessment of sensitivity to HFIP in each cell was performed by cell staining using crystal violet. The crystal violet is a reagent that binds to the cell membrane of living cells and stains them purple. After washing once with PBS (phosphate buffered saline), 100% methanol was added, and the cells were fixed by incubating at room temperature for 15 minutes. Then, methanol was removed, a 0.05% (weight / volume%) crystal violet solution (Fujifilm Wako Pure Chemical Corporation) was added, and the cells were stained by standing at room temperature for 15 minutes. After removing the crystal violet solution with a pipette, the number of living cells was assessed from the stained area per well. A plate appearance photograph of Example 1 after cell staining is shown at the lower stage of FIG. 1, and a plate appearance photograph of Comparative Example 1 is shown at the upper stage of FIG. 1.

As shown in FIG. 1, the upper concentration limit of HFIP at which N2a cells can survive was 25 mM, while the upper concentration limit at which ScN2a cells can survive was 20 mM. This result indicates that N2a cells and ScN2a cells have different sensitivity to HFIP. This difference in sensitivity to HFIP has shown that HFIP can be used to detect an abnormally structured prion protein. Therefore, it has been shown that it can be suitably used for simple screening and diagnosis of a protein misfolding disease with the β-sheet structure and amyloid structure.

### <Assessment of protease resistance>

A 6-well plate for cell culture was seeded with 3 × 10⁵ of cells per well, and N2a cells and ScN2a cells were incubated in E-MEM at 37°C under 5% CO₂ for 2 days.

### [Example 2]

The medium of ScN2a cells was replaced with a medium with a final concentration of HFIP of 6 mM and incubated for 24 hours.

### [Comparative Example 2]

The procedure was carried out in the same manner as in Example 2, except that the cells used were changed to N2a cells.

The medium was removed by suction after 24 hours of incubation, and each cell was washed with cooled PBS. Then, 500 µl of cooled RIPA buffer (Fujifilm Wako Pure Chemical Corporation) was added per well, and the cells were lysed by standing on ice for 1 minute. The total amount of cytolytic solution was collected in a 1.5 ml tube and centrifuged at 4°C, 3000 rpm for 10 minutes. 200 µl of the centrifuged supernatant was transferred into a new tube, and protease K (Proteinase K, NACALAI TESQUE, INC.) was added to a final concentration of 10 µg/ml, and the mixture was reacted in a constant temperature bath at 37°C for 30 minutes. Then, Protease inhibitor (NACALAI TESQUE, INC.) was added, and the protease K activity was deactivated by reacting at room temperature for 5 minutes using a tube rotor.

Next, the mixture was centrifuged at 4°C, 13000 rpm for 20 minutes, and the supernatant was removed by a pipette. To the sedimented fraction, 20 µl of lysis buffer for gel electrophoresis (150 mM Tris-HCI (Tris hydrochloride buffer) pH 6.8, 6% SDS (sodium dodecyl sulfate), 30% glycerol, 0.01% BPB (bromophenol blue), and 100 mM DTT (dithiothreitol)) were added, incubated at 95°C for 5 minutes, and subjected to SDS-PAGE (polyacrylamide gel electrophoresis).

A Western blotting method was used to detect a protease K-resistance prion protein. Samples were run on a Tris-Glycine SDS Running Buffer (25 mM Tris, 192 mM Glycine, 0.1% SDS) at 100 V for 15 minutes using 15% acrylamide gel (DRC CO., LTD.), and proteins were separated according to the molecular weight.

The gel after running was transferred to a PVDF membrane (polyvinylidene fluoride). iBlot2 Transfer Stacks, PVDF, regular size (Cat. IB24001) was used as the PVDF membrane. Transfer was carried out using iBlot Gel Transfer Device (Thermo Fisher Scientific K. K.) and energized at 20 V for 1 minute, at 23 V for 4 minutes, and at 25 V for 2 minutes for a total of 7 minutes. The PVDF membrane after the transfer was treated in PBS-Tween20 (PBS-T) with 5% (weight / volume%) skim milk for 30 minutes at room temperature (blocking), and anti-PrP antibody diluted 1000-fold with PBS-T (Cat. A03207, SPI-Bio) was added to the PVDF membrane, and incubated overnight at room temperature. After washing the PVDF membrane with PBS-T with shaking once in 15 minutes and three times in 5 minutes, HRP-labeled anti-mouse IgG (Cat. W4021, Promega) diluted 5000-fold with PBS-T was added and incubated with shaking for 1 hour at room temperature. Thereafter, the PVDF membrane was washed with PBS-T while shaking once in 15 minutes and three times in 5 minutes, and then a chemiluminescent reagent (Immobilon Western chemiluminescent HRP substrate, Merk Millipore) was added to the PVDF membrane and incubated for 1 minute, and then a protease K-resistance prion protein was detected by an image analyzer (ChemiDoc Touch, Bio-Rad Laboratories, Hercules).

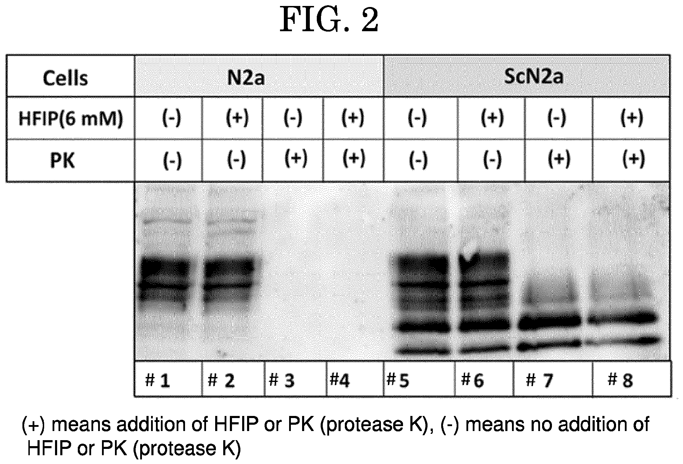

The results of Western blotting of Example 2 are shown in #5 to #8 of FIG. 2, and the results of Western blotting of Comparative Example 2 are shown in #1 to #4 of FIG. 2.

As shown in FIG. 2, it was shown that the protein extracted from N2a cells of Comparative Example 2 was degraded by protease K with or without the addition of HFIP (#1 to #4). However, the protein extracted from ScN2a cells of Example 2 showed a change in the band after acting on Protease K with or without the addition of HFIP. This indicates that resistance to protease K was reduced in ScN2a cells added with HFIP. Therefore, it was suggested that HFIP has the activity of alleviating abnormal structures of PrP^{Sc} in ScN2a cells.

### [Industrial Applicability]

A method for selectively killing protein aggregate-containing cells in the present invention and a kit thereof are usefully used for diagnostic applications of a protein misfolding disease. It is also applicable to the treatment of a protein misfolding disease.

### REFERENCES SIGNS LIST

100: inspection apparatus, 101: input unit, 120: detection unit, 130: storage unit, 140: display unit, 150: control unit, 160: calculation unit, 170: power supply device, 180: communication unit, 200: network, 300: server, 400: terminal

## Claims

1. A method for selectively killing protein aggregate-containing cells comprising:
treating cells with a molecular structure-modifying agent, the molecular structure-modifying agent containing a fluorine-based alcohol or a compound represented by general formula (1), 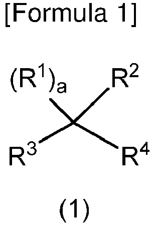
wherein, in general formula (1), a is 0 or 1,
when a is 1, then R¹ is a hydrogen atom and R² is a hydroxyl group, or R¹ is a hydroxyl group and R² is a hydrogen atom,
when a is 0, then R² is an oxygen atom forming a double bond together with a carbon atom,
R³ is CHₗClₘFₙ, and I is an integer from 0 to 3, m and n are integers from 1 to 3, and I + m + n = 3 is satisfied,
R⁴ is CHₛClₜFᵤ, and s is an integer from 0 to 3, t and u are integers from 1 to 3, and s + t + u = 3 is satisfied.
I + s < 6 is satisfied.

2. The method for selectively killing protein aggregate-containing cells according to claim 1, wherein
the fluorine-based alcohol is presented by a general formula RfCH₂OH or RfRf'CHOH,
Rf and Rf' present a perfluoroalkyl group having 1 to 10 carbon atoms, and
Rf and Rf' are different from each other or the same.

3. The method for selectively killing protein aggregate-containing cells according to claim 1, wherein the fluorine-based alcohol is 1,1,1,3,3,3-hexafluoro-2-propanol.

4. The method for selectively killing protein aggregate-containing cells according to claim 1, wherein a concentration of the fluorine-based alcohol or a compound represented by general formula (1) when treating the cells is in a range from 1 pM to 100 mM.

5. A kit for selectively killing protein aggregate-containing cells comprising:
a fluorine-based alcohol or a compound represented by general formula (1), 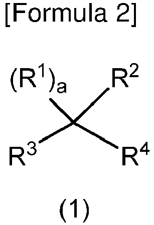
wherein, in general formula (1), a is 0 or 1,
when a is 1, then R¹ is a hydrogen atom and R² is a hydroxyl group, or R¹ is a hydroxyl group and R² is a hydrogen atom,
when a is 0, then R² is an oxygen atom forming a double bond together with a carbon atom,
R³ is CHₗClₘFₙ, and I is an integer from 0 to 3, m and n are integers from 1 to 3, and I + m + n = 3 is satisfied,
R⁴ is CHₛClₜFᵤ, and s is an integer from 0 to 3, t and u are integers from 1 to 3, and s + t + u = 3 is satisfied.
I + s < 6 is satisfied.

6. The kit for selectively killing protein aggregate-containing cells according to claim 5, wherein
the fluorine-based alcohol is presented by a general formula RfCH₂OH or RfRf'CHOH,
Rf and Rf' present a perfluoroalkyl group having 1 to 10 carbon atoms, and
Rf and Rf' are different from each other or the same.

7. The kit for selectively killing protein aggregate-containing cells according to claim 1, wherein the fluorine-based alcohol is 1,1,1,3,3,3-hexafluoro-2-propanol.

8. The kit for selectively killing protein aggregate-containing cells according to claim 5, wherein a concentration of the fluorine-based alcohol or a compound represented by general formula (1) when treating the cells is in a range from 1 pM to 100 mM.

9. A therapeutic agent for a protein misfolding disease comprising:
a fluorine-based alcohol or a compound represented by general formula (1), 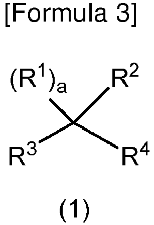
wherein, in general formula (1), a is 0 or 1,
when a is 1, then R¹ is a hydrogen atom and R² is a hydroxyl group, or R¹ is a hydroxyl group and R² is a hydrogen atom,
when a is 0, then R² is an oxygen atom forming a double bond together with a carbon atom,
R³ is CHₗClₘFₙ, and I is an integer from 0 to 3, m and n are integers from 1 to 3, and I + m + n = 3 is satisfied,
R⁴ is CHₛClₜFᵤ, and s is an integer from 0 to 3, t and u are integers from 1 to 3, and s + t + u = 3 satisfied.
I + s < 6 is satisfied.

10. The therapeutic agent for the protein misfolding disease according to claim 9, wherein
the fluorine-based alcohol is presented by a general formula RfCH₂OH or RfRf'CHOH,
Rf and Rf' present a perfluoroalkyl group having 1 to 10 carbon atoms, and
Rf and Rf' are different from each other or the same.

11. The therapeutic agent for the protein misfolding disease according to claim 9, wherein the fluorine-based alcohol is 1,1,1,3,3,3-hexafluoro-2-propanol.

12. An agent for removing a protein aggregate from a blood derivative comprising:
a fluorine-based alcohol or a compound represented by general formula (1), 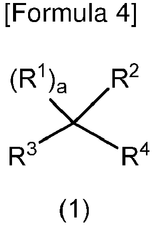
wherein, in general formula (1), a is 0 or 1,
when a is 1, then R¹ is a hydrogen atom and R² is a hydroxyl group, or R¹ is a hydroxyl group and R² is a hydrogen atom,
when a is 0, then R² is an oxygen atom forming a double bond together with a carbon atom,
R³ is CHₗClₘFₙ, and I is an integer from 0 to 3, m and n are integers from 1 to 3, and I + m + n = 3 is satisfied,
R⁴ is CHₛClₜFᵤ, and s is an integer from 0 to 3, t and u are integers from 1 to 3, and satisfied that s + t + u = 3 is satisfied.
I + s < 6 is satisfied.

13. The agent for removing the protein aggregate from the blood derivative according to claim 12, wherein
the fluorine-based alcohol is presented by a general formula RfCH₂OH or RfRf'CHOH,
Rf and Rf' present a perfluoroalkyl group having 1 to 10 carbon atoms, and
Rf and Rf' are different from each other or the same.

14. The agent for removing the protein aggregate from the blood derivative according to claim 12, wherein the fluorine-based alcohol is 1,1,1,3,3,3-hexafluoro-2-propanol.
